# EUROPÄISCHE PATENTSCHRIFT

(11) **EP 4 265 222 B1**
(45) Veröffentlichungstag und Bekanntmachung des Hinweises auf die Patenterteilung: **18.09.2024**
(21) Anmeldenummer: 23166902.9
(22) Anmeldetag: 06.04.2023
(51) Int. Cl.: A61F 2/16

(54) **INJEKTOR MIT GRIFF ZUM MITNEHMEN EINES VORDEREN UND EINES HINTEREN VERLAGERUNGSMECHANISMUS**
INJECTOR WITH HANDLE FOR ACCOMMODATING A FRONT AND A REAR DISPLACEMENT MECHANISM
INJECTEUR DOTÉ D'UNE POIGNÉE POUR RECEVOIR UN MÉCANISME DE DÉPLACEMENT AVANT ET ARRIÈRE

(30) Priorität: 21.04.2022 DE 102022109691
(43) Veröffentlichungstag der Anmeldung: 25.10.2023
(73) Patentinhaber: Carl Zeiss Meditec AG, 07745 Jena (DE)
(72) Erfinder: KELP, Martin, 73447 Oberkochen (DE); HÖLZEL, Marco, 73447 Oberkochen (DE); MOEIN, Hadi, 73447 Oberkochen (DE)
(74) Vertreter: PATERIS Patentanwälte PartmbB

(56) Entgegenhaltungen:
- EP-A1- 2 386 272
- US-A1- 2011 276 054
- US-A1- 2014 257 317
- US-A1- 2015 342 726

## Beschreibung

Die Erfindung betrifft einen Injektor für ein Einführen einer Intraokularlinse in den Kapselsack eines Auges.

Bei einer Kataraktbehandlung eines Auges wird herkömmlich nur ein kleiner Schnitt in die Hornhaut des Auges eingebracht, der so groß ist, dass eine Spitze eines Injektors durch den Schnitt in das Auge eingeführt werden kann. Nachdem der Schnitt in die Hornhaut eingebracht wurde, wird die Linse des Auges beispielsweise mittels Phakoemulsifikation zerkleinert und anschließend aus dem Kapselsack des Auges abgesaugt. Danach wird eine Intraokularlinse mittels des Injektors in das Auge eingesetzt. Die Intraokularlinse wird in dem Injektor gefaltet, so dass sie durch die Spitze passt. Die Spitze wird durch den Schnitt in den Kapselsack eingeführt und die gefaltete Intraokularlinse wird mittels des Injektors durch die Spitze in den Kapselsack geschoben, in dem die Intraokularlinse sich entfaltet und somit die ursprüngliche Linse ersetzt. Während die Intraokularlinse gefaltet wird oder während die gefaltete Intraokularlinse in den Kapselsack geschoben wird, kann es zu Fehlern kommen. Die Fehler können beispielsweise dazu führen, dass sich die Intraokularlinse nicht vollständig in dem Kapselsack entfaltet oder dass die Intraokularlinse sogar beschädigt wird. EP 02 386 272 A1 offenbart eine Einführungsvorrichtung für eine Intraokularlinse. DE 10 2021 116 615 B3 offenbart einen Injektor mit einem ersten Kolben und einem zweiten Kolben. US 2014/0257317 A1 offenbart einen automatisierten vorgeladenen Intraokularlinseninjektor. US 2015/0342726 A1 offenbart eine Einführapparatur für ein Okularimplantat. US 2011/0276054 A1 offenbart ein Einführsystem für eine IOL, mit einem halbautomatischen Konfigurationsmanagement für die hintere Haptik.

Aufgabe der Erfindung ist es daher, einen Injektor zu schaffen, mit der eine Wahrscheinlichkeit gering ist, dass Fehler bei einer Benutzung des Injektors auftreten.

Der erfindungsgemäße Injektor für ein Einführen einer Intraokularlinse in den Kapselsack eines Auges weist einen Injektorkörper, der einen Innenraum begrenzt, in dem die Intraokularlinse in einem Lagerungszustand der Intraokularlinse anzuordnen ist, eine Injektorspitze, die eine Öffnung aufweist, und einen Kolben auf, der eingerichtet ist, die Intraokularlinse durch ein Längsverlagern des Kolbens in einer Einführrichtung hin zu der Öffnung via die Öffnung aus dem Injektor heraus zu verlagern. Die Intraokularlinse weist einen Optikkörper mit einer optischen Achse und eine auf die Einführrichtung bezogene hintere Haptik und eine auf die Einführrichtung bezogene vordere Haptik auf. Der Injektor weist zudem einen hinteren Verlagerungsmechanismus, der eingerichtet ist, durch ein Verlagern des Kolbens ein dem Optikkörper abgewandtes Längsende der hinteren Haptik entlang einer hinteren Trajektorie auf den Optikkörper zu verlagern, und einen vorderen Verlagerungsmechanismus auf, der eingerichtet ist, ein dem Optikkörper abgewandtes Längsende der vorderen Haptik entlang einer vorderen Trajektorie zu verlagern, die eine vordere Komponente hat, die parallel zu der optischen Achse ist. Des Weiteren weist der Injektor einen Griff auf, der eingerichtet ist, von außerhalb des Injektors bedienbar zu sein. Der Griff weist einen ersten Mitnehmer, der mit dem Kolben gekoppelt ist und eingerichtet ist, durch ein Längsverlagern des Griffs den Kolben in der Einführrichtung mitzunehmen, und einen zweiten Mitnehmer auf, der eingerichtet ist, den vorderen Verlagerungsmechanismus mitzunehmen, so dass durch das Längsverlagern des Griffs das Längsende der hinteren Haptik auf den Optikkörper verlagerbar ist, das Längsende der vorderen Haptik entlang der vorderen Trajektorie verlagerbar ist und der Optikkörper mittels des Kolbens in der Einführrichtung verlagerbar ist.

Dadurch, dass der Griff sowohl den ersten Mitnehmer als auch den zweiten Mitnehmer aufweist, kann es nicht vergessen werden, das Längsende der vorderen Haptik und/oder das Längsende der hinteren Haptik auf den Optikkörper zu verlagern. Indem das Längsende der hinteren Haptik und das Längsende der vorderen Haptik durch das Längsverlagern des Griffs auf den Optikkörper angeordnet werden, werden das Längsende der vorderen Haptik und das Längsende der hinteren Haptik innerhalb des Optikkörpers angeordnet, wenn die Intraokularlinse von dem Kolben in die Injektorspitze verlagert wird und dadurch gefaltet wird. Eine Beschädigung der vorderen Haptik und der hinteren Haptik ist dadurch unwahrscheinlich. Damit kann ein fehlerarmes und reproduzierbares Einbringen der Intraokularlinse in den Kapselsack eines Auges und ein fehlerarmes und reproduzierbares Entfalten der Intraokularlinse in dem Kapselsack erreicht werden.

Der vordere Verlagerungsmechanismus weist bevorzugt eine Auflagewand, auf der in dem Lagerungszustand das Längsende der vorderen Haptik aufliegt und die das Längsende der vorderen Haptik in der Richtung der vorderen Trajektorie abstützt, und eine erste Seitenwand auf, die eingerichtet ist, dass die vordere Haptik an die erste Seitenwand anstößt, wenn die vordere Haptik in der Einführrichtung verlagert. Durch ein Verlagern der Auflagewand ist es möglich, das Längsende der vorderen Haptik entlang der vorderen Trajektorie zu verlagern. Indem die vordere Haptik an die erste Seitenwand anstößt, kann ein Verlagern der vorderen Haptik in der Einführrichtung verzögert werden, wenn der Optikkörper verlagert, so dass das Längsende der vorderen Haptik auf den Optikkörper gelangen kann.

Es ist bevorzugt, dass der vordere Verlagerungsmechanismus eingerichtet ist, die Auflagewand so weit entlang der vorderen Trajektorie zu verlagern, dass bei einem Verlagern des Optikkörpers in der Einführrichtung der Optikkörper auf eine dem Längsende der vorderen Haptik abgewandten Seite der Auflagewand gelangt.

Es ist bevorzugt, dass der vordere Verlagerungsmechanismus eingerichtet ist, das Längsende der vorderen Haptik so weit entlang der vorderen Trajektorie zu verlagern, dass bei einem Verlagern des Optikkörpers in der Einführrichtung das Längsende der vorderen Haptik auf den Optikkörper verlagert.

Der vordere Verlagerungsmechanismus weist bevorzugt eine Schwenkvorrichtung auf, die eine Achse aufweist, die drehbar an dem Injektorkörper gelagert ist, so dass die vordere Trajektorie im Wesentlichen eine Form eines Kreisbogens hat. Die vordere Trajektorie hat im Wesentlichen die Form des Kreisbogens, weil sich eine geringfügige Abweichung der vorderen Trajektorie von der Kreisform dadurch ergeben kann, dass sich das Längsende bei einer Drehung der Schwenkvorrichtung geringfügig relativ zu der Schwenkvorrichtung verlagern kann. Es ist besonders bevorzugt, dass die Schwenkvorrichtung die Auflagewand und die erste Seitenwand aufweist.

Alternativ bevorzugt weist der vordere Verlagerungsmechanismus einen Mechanismusgleiter auf, der längsverlagerbar an dem Injektorkörper gelagert ist, so dass die vordere Trajektorie im Wesentlichen gerade ist. Die vordere Trajektorie ist im Wesentlichen gerade, weil sich eine geringfügige Abweichung einer geraden Form dadurch ergeben kann, dass sich das Längsende bei einer Verlagerung des Mechanismusgleiters geringfügig relativ zu dem Mechanismusgleiter verlagern kann. Besonders bevorzugt ist der Mechanismusgleiter parallel zu der optischen Achse verlagerbar, wodurch die vordere Trajektorie im Wesentlichen parallel zu der optischen Achse orientiert ist.

Der hintere Verlagerungsmechanismus weist bevorzugt eine Gleitfläche auf, an der das Längsende der hinteren Haptik eingerichtet ist, entlang zu gleiten, wenn der Kolben in der Einführrichtung verlagert, und die so gegen die Einführrichtung geneigt angeordnet ist, dass dabei die hintere Trajektorie eine hintere Komponente parallel zu der optischen Achse hat. Dabei ist besonders bevorzugt, dass die hintere Komponente in der gleichen Richtung wie die vordere Komponente orientiert ist. Dadurch verlagern das Längsende der vorderen Haptik und das Längsende der hinteren Haptik auf die gleiche Seite des Optikkörpers.

Der Kolben weist bevorzugt einen Stößel, der eingerichtet ist, den Optikkörper zu verlagern, und einen ersten Gleiter auf, der ein Teil des hinteren Verlagerungsmechanismus ist und eingerichtet ist, die hintere Haptik in einem Bereich des Längsendes der hinteren Haptik zu kontaktieren und somit auf den Optikkörper zu verlagern. Durch das Vorsehen des ersten Gleiters kann vermieden werden, dass die hintere Haptik in einem Bereich des Längsendes der hinteren Haptik entgegen die Einführrichtung abknickt und das Längsende der hinteren Haptik somit nicht auf den Optikkörper verlagert werden kann. Dadurch ist der Injektor besonders fehlerarm.

Der Injektorkörper weist bevorzugt eine Seitenfläche auf, die den Innenraum begrenzt, gegen die der erste Gleiter vorgespannt ist, an der der erste Gleiter entlang gleitet, wenn der Kolben in der Einführrichtung verlagert, und die so gegen die Einführrichtung geneigt ist, dass mit zunehmender Verlagerung des Kolbens in der Einführrichtung der erste Gleiter das Längsende der hinteren Haptik näher zu der optischen Achse verlagert. Dadurch wird erreicht, dass mit zunehmender Verlagerung des Kolbens in der Einführrichtung der erste Gleiter die Krümmung der hinteren Haptik vergrößert.

Der Stößel weist bevorzugt an seinem dem Optikkörper zugewandten Ende eine Stößelausnehmung auf, die eingerichtet ist, die hintere Haptik und den Optikkörper aufzunehmen. Dadurch kann vermieden werden, dass die Intraokularlinse an dem Kolben vorbei rutscht, wodurch der Kolben die Intraokularlinse nicht aus dem Injektor heraus verlagern könnte.

Es ist bevorzugt, dass der Stößel einen ersten Zahn des Stößels aufweist, wobei der erste Zahn des Stößels eine Ansteigeflanke des Stößels aufweist, an der die hintere Haptik eingerichtet ist, entlang zu gleiten, wenn der Kolben in der Einführrichtung verlagert wird. Die Ansteigeflanke ist so gegen die Einführrichtung geneigt angeordnet, dass die hintere Haptik im Bereich der Stößelausnehmung parallel zu der optischen Achse und in der gleichen Richtung wie die hintere Komponente verlagert. Die Ansteigeflanke unterstützt somit das Verlagern des Längsendes der hinteren Haptik auf den Optikkörper.

Der Stößel weist bevorzugt einen zweiten Zahn des Stößels auf, wobei der zweite Zahn des Stößels die Stößelausnehmung an einer der Ansteigeflanke abgewandten Seite der Stößelausnehmung begrenzt.

Der Kolben weist bevorzugt eine Gleiteranordnung auf, die den ersten Gleiter und eine Stößelaufnahme aufweist, in der der Stößel längsverlagerbar relativ zu der Gleiteranordnung angeordnet ist, wobei die Gleiteranordnung mit dem ersten Mitnehmer gekoppelt ist, wodurch der erste Mitnehmer mit dem Kolben gekoppelt ist. Die Gleiteranordnung weist einen Anschlag auf und der Stößel weist eine Stößelverdickung auf, die eingerichtet ist, gegen den Anschlag anzuschlagen, wenn der Stößel entgegen der Einführrichtung relativ zu der Gleiteranordnung verlagert. Damit sind der Anschlag und die Stößelverdickung eingerichtet, ein Längsverlagern des Stößels relativ zu der Gleiteranordnung entgegen der Einführrichtung zu begrenzen. Dadurch ist die Gleiteranordnung eingerichtet, den Stößel mitzunehmen und somit in der Einführrichtung zu verlagern, wenn der Griff verlagert wird. Anschließend kann durch ein Drücken des Stößels in Richtung zu der Öffnung hin der Stößel weiter in Richtung zu der Öffnung verlagert werden. Weil der Stößel längsverlagerbar relativ zu der Gleiteranordnung angeordnet ist, kann die Gleiteranordnung dabei an Ort und Stelle verbleiben, wodurch es nicht erforderlich ist, die Gleiteranordnung ebenfalls in die Injektorspitze hinein zu verlagern.

Die Gleiteranordnung weist bevorzugt eine Mitnehmeraufnahme auf, in die der erste Mitnehmer eingreift, um den ersten Mitnehmer mit der Gleiteranordnung zu koppeln. Dadurch kann der erste Mitnehmer lösbar mit dem Kolben gekoppelt sein. Indem der erste Mitnehmer aus der Mitnehmeraufnahme entfernt wird, kann der erste Mitnehmer besonders einfach von dem Kolben entkoppelt werden, wenn das Längsende der vorderen Haptik und das Längsende der hinteren Haptik auf den Optikkörper verlagert sind.

Die vordere Haptik und/oder die hintere Haptik sind bevorzugt C-förmig oder J-förmig.

Es ist bevorzugt, dass der Injektor die Intraokularlinse aufweist, die in dem Lagerungszustand in dem Innenraum angeordnet ist.

Im Folgenden wird anhand der beigefügten schematischen Zeichnungen die Erfindung näher erläutert. Es zeigen
Figur 1 eine Draufsicht auf eine erste Ausführungsform eines erfindungsgemäßen Injektors zu einem ersten Zeitpunkt,
Figur 2 einen Schnitt A-A aus Figur 1,
Figur 3 einen Ausschnitt aus Figur 2,
Figur 4 die Figur 3 zu einem vierten Zeitpunkt,
Figur 5 eine Draufsicht auf eine zweite Ausführungsform des erfindungsgemäßen Injektors zu einem ersten Zeitpunkt,
Figur 6 die Figur 5 zu einem zweiten Zeitpunkt,
Figur 7 die Figur 5 zu einem dritten Zeitpunkt,
Figur 8 die Figur 5 zu dem vierten Zeitpunkt,
Figur 9 die Figur 5 zu einem fünften Zeitpunkt,
Figur 10 einen Schnitt B-B aus Figur 5,
Figur 11 einen Schnitt C-C aus Figur 8,
Figur 12 einen Längsschnitt durch eine erste Ausführungsform eines Griffs des Injektors,
Figur 13 einen Längsschnitt durch eine dritte Ausführungsform des Injektors mit einer zweiten Ausführungsform des Griffs,
Figur 14 eine perspektivische Ansicht einer Gleiteranordnung des Injektors,
Figur 15 eine Draufsicht auf einen Stößel des Injektors,
Figur 16 eine perspektivische Ansicht eines Abschnitts einer vierten Ausführungsform des Injektors,
Figur 17 eine perspektivische Ansicht eines Abschnitts einer fünften Ausführungsform des Injektors,
Figur 18 eine perspektivische Ansicht eines Abschnitts einer sechsten Ausführungsform des Injektors,
Figur 19 einen Längsschnitt durch die sechste Ausführungsform,
Figur 20 eine Draufsicht auf eine siebte Ausführungsform des Injektors und
Figur 21 eine Seitenansicht eines zweiten Gleiters des Injektors,
Figur 22 einen Längsschnitt durch eine achte Ausführungsform des Injektors und
Figur 23 eine perspektivische Ansicht der achten Ausführungsform.

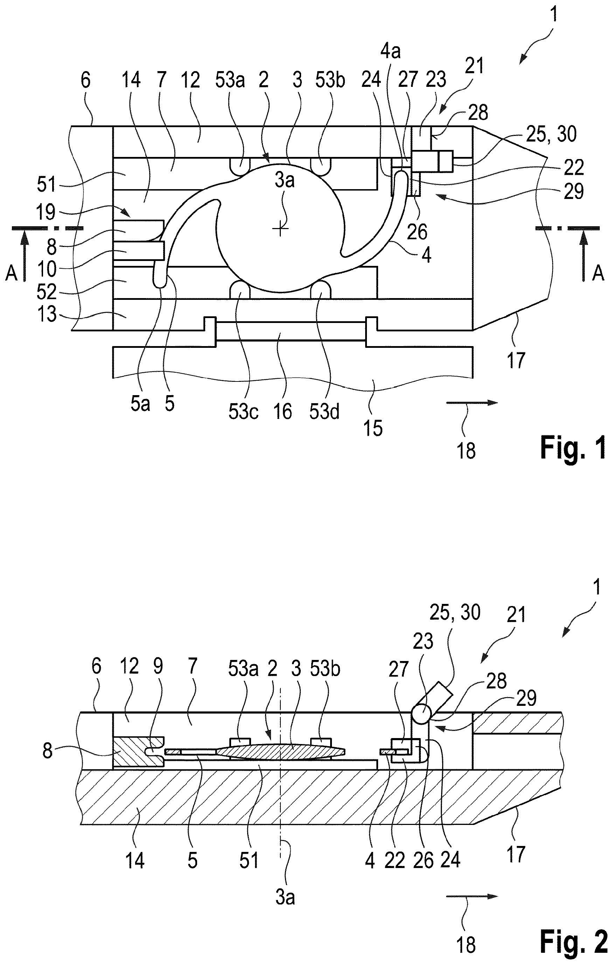

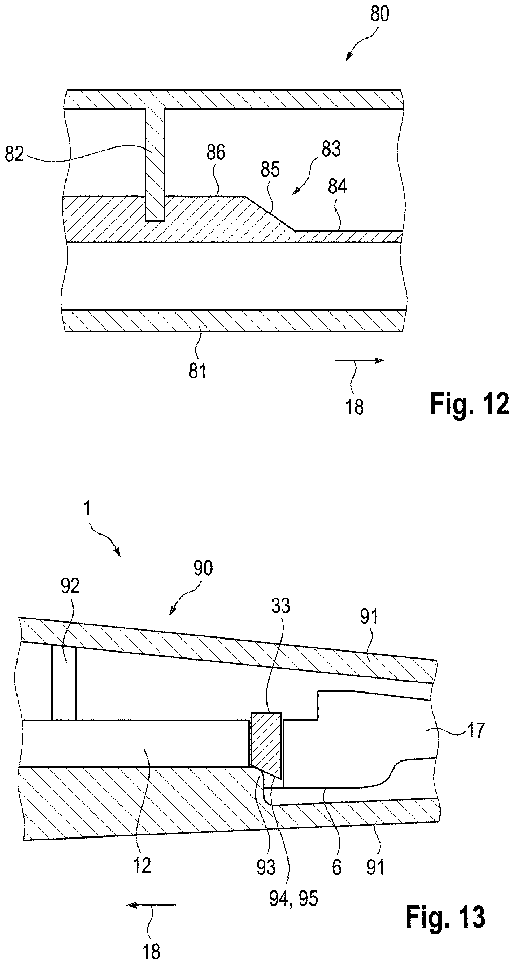

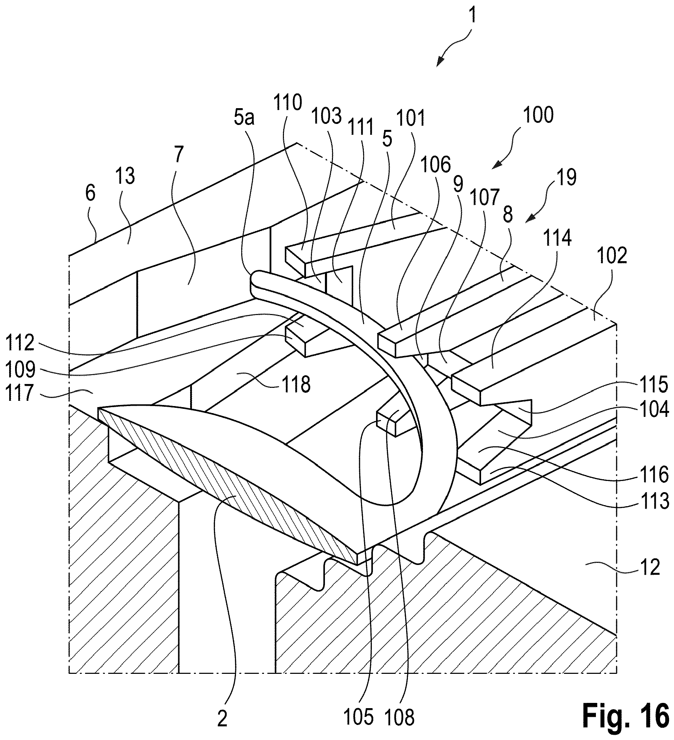

Wie es aus Figuren 1 bis 11 und 16 bis 18 ersichtlich ist, weist ein Injektor 1 für ein Einführen einer Intraokularlinse 2 in den Kapselsack eines Auges einen Injektorkörper 6, der einen Innenraum 7 begrenzt, in dem die Intraokularlinse 2 in einem Lagerungszustand der Intraokularlinse 2 anzuordnen ist, eine Injektorspitze 17, die eine Öffnung 20 aufweist, und einen Kolben 19 auf, der eingerichtet ist, die Intraokularlinse 2 durch ein Längsverlagern des Kolbens 19 in einer Einführrichtung 18 hin zu der Öffnung 20 via die Öffnung 20 aus dem Injektor 1 heraus zu verlagern. Die Intraokularlinse 2 weist einen Optikkörper 3 mit einer optischen Achse 3a, eine auf die Einführrichtung 18 bezogene hintere Haptik 5 und eine auf die Einführrichtung 18 bezogene vordere Haptik 4 auf. Zudem weist der Injektor 1 einen hinteren Verlagerungsmechanismus 100, 130, 160 auf (siehe Figuren 16 bis 19), der eingerichtet ist, durch ein Verlagern des Kolbens 19 ein dem Optikkörper 3 abgewandtes Längsende 5a der hinteren Haptik 5 entlang einer hinteren Trajektorie auf den Optikkörper 3 zu verlagern. Des Weiteren weist der Injektor 1 einen vorderen Verlagerungsmechanismus 21, 31 auf (siehe Figuren 1 bis 11), der eingerichtet ist, ein dem Optikkörper 3 abgewandtes Längsende 4a der vorderen Haptik 4 entlang einer vorderen Trajektorie zu verlagern, die eine vordere Komponente hat, die parallel zu der optischen Achse 3a ist. Der Injektor 1 weist einen Griff 80, 90, 96 auf (siehe Figuren 12, 13, 22 und 23), der eingerichtet ist, von außerhalb des Injektors 1 bedienbar zu sein. Der Griff 80, 90, 96 weist einen ersten Mitnehmer 82, 92, 97, der mit dem Kolben 19 gekoppelt ist und eingerichtet ist, durch ein Längsverlagern des Griffs 80, 90, 96 den Kolben 19 in der Einführrichtung 18 mitzunehmen, und einen zweiten Mitnehmer 83, 93 auf, der eingerichtet ist, den vorderen Verlagerungsmechanismus 21, 31 mitzunehmen, so dass durch das Längsverlagern des Griffs 80, 90, 96 das Längsende 5a der hinteren Haptik 5 auf den Optikkörper 3 verlagerbar ist, das Längsende 4a der vorderen Haptik 4 entlang der vorderen Trajektorie verlagerbar ist und der Optikkörper 3 mittels des Kolbens 19 in der Einführrichtung 18 verlagerbar ist.

Der Injektor 1 kann die Intraokularlinse 2 aufweisen, die in dem Lagerungszustand in dem Innenraum 7 angeordnet ist.

Die vordere Trajektorie und hintere Trajektorie können beispielsweise relativ zu dem Injektorkörper 6 verlaufen.

Figuren 1 und 5 bis 9 zeigen, dass die vordere Haptik 4 und die hintere Haptik 5 C-förmig sein können. In einem anderen Beispiel sind die vordere Haptik 4 und die hintere Haptik 5 J-förmig.

Insbesondere aus Figuren 1 und 5 bis 9 ist ersichtlich, dass der Injektor 1 einen ersten Haltevorsprung 53a und einen zweiten Haltevorsprung 53b, die an der ersten Injektorkörperseitenwand 12 befestigt sind und in den Innenraum 7 hinein vorstehen, und einen dritten Haltevorsprung 53c und einen vierten Haltevorsprung 53d aufweisen kann, die an der zweiten Injektorkörperseitenwand 13 befestigt sind und in den Innenraum 7 hinein vorstehen. Der Optikkörper 3 kann in dem Lagerungszustand von den Haltevorsprüngen 53a, 53b, 53c, 53d eingeklemmt sein. Zudem kann der Injektor 1 ein erstes Podest 51, das den ersten Haltevorsprung 53a und den zweiten Haltevorsprung 53b abstützt, und ein zweites Podest 52 aufweisen, das den dritten Haltevorsprung 53c und den vierten Haltevorsprung 53d abstützt.

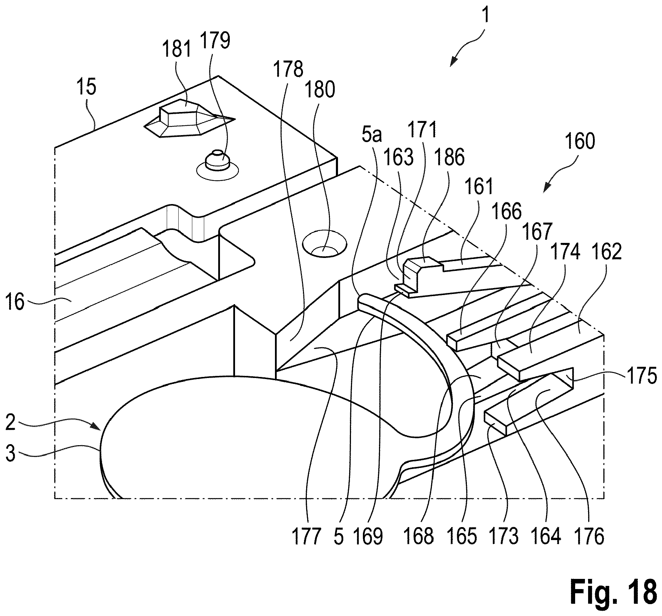

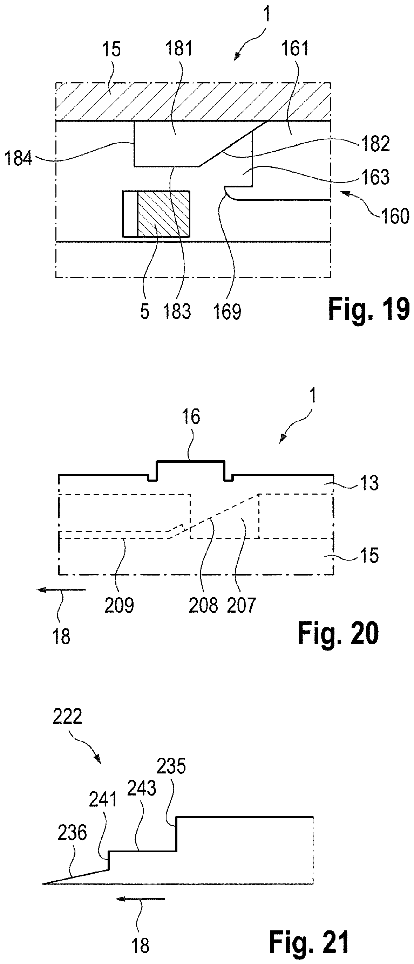

Insbesondere Figuren 1 bis 11 zeigen, dass der Injektorkörper 6 eine erste Injektorkörperseitenwand 12 und eine zweite Injektorkörperseitenwand 13 aufweisen kann, die den Innenraum 7 an einander abgewandten Seiten des Innenraums 7 begrenzen können. Dabei können in dem Lagerungszustand das Längsende 4a der vorderen Haptik 4 der ersten Injektorkörperseitenwand 12 zugewandt angeordnet sein und das Längsende 5a der hinteren Haptik 5 der zweiten Injektorkörperseitenwand 13 zugewandt angeordnet sein, vergleiche Figuren 1 und 5 bis 9. Zudem kann das Längsende 4a der vorderen Haptik 4 näher an der ersten Injektorkörperseitenwand 12 als an der zweiten Injektorkörperseitenwand 13 angeordnet sein und kann das Längsende 5a der hinteren Haptik 5 näher an der zweiten Injektorkörperseitenwand 13 als an der ersten Injektorkörperseitenwand 12 angeordnet sein. Zudem kann der Injektorkörper 6 einen Injektorkörperboden 14 und einen Deckel 15 (vergleiche Figuren 1 und 18 bis 20) aufweisen, die den Innenraum 7 an einander abgewandten Seiten des Innenraums 7 begrenzen. Die optische Achse 3a kann im Wesentlichen senkrecht zu dem Injektorkörperboden 14 angeordnet sein. Der Deckel 15 kann mittels eines Deckelgelenks 16 verschwenkbar zu dem verbliebenen Injektorkörper 6 angeordnet sein. Der Deckel 15 kann dadurch einen Offenzustand (vergleiche Figuren 1 und 18), in dem der Innenraum 7 zugänglich ist, und einen Geschlossenzustand (vergleiche Figuren 19 und 20) aufweisen, in dem der Innenraum 7 nicht zugänglich ist und der Deckel 15 den Innenraum 7 begrenzt.

Figuren 1 bis 4 und 13 zeigen, dass der vordere Verlagerungsmechanismus 21, 31 einen Verlagerungsmechanismusanschlag 30, 95 aufweisen kann, der eingerichtet ist, dass der zweite Mitnehmer 83, 93 gegen den Verlagerungsmechanismusanschlag 30, 95 anschlägt, wenn der Griff 80, 90, 96 in die Einführrichtung 18 verlagert wird. Der zweite Mitnehmer 83, 93 ist eingerichtet, bei einem weiteren Verlagern des Griffs 80, 90, 96 den Verlagerungsmechanismusanschlag 30, 95 zu verlagern und somit den vorderen Verlagerungsmechanismus 21, 31 mitzunehmen.

Figuren 1 bis 11 zeigen, dass der vordere Verlagerungsmechanismus 21, 31 eine Auflagewand 22, 32, auf der in dem Lagerungszustand das Längsende 4a der vorderen Haptik 4 aufliegt und die das Längsende 4a der vorderen Haptik 4 in der Richtung der vorderen Trajektorie abstützt, und eine erste Seitenwand 26, 36 aufweisen kann, die eingerichtet ist, dass die vordere Haptik 4 an die erste Seitenwand 26, 36 anstößt, wenn die vordere Haptik 4 in der Einführrichtung 18 verlagert. Zudem kann der vordere Verlagerungsmechanismus 21, 31 eine zweite Seitenwand 27, 37 aufweisen, die ein Verlagern des Längsendes 4a der vorderen Haptik 4 in einer Richtung senkrecht zu der Einführrichtung 18 und senkrecht zu der optischen Achse 3a weg von der optischen Achse 3a begrenzt.

In Figuren 1 bis 4 ist dargestellt, dass der vordere Verlagerungsmechanismus 21 eine Schwenkvorrichtung 29 aufweisen kann, die eine Achse 23 aufweist, die drehbar beispielsweise an dem Injektorkörper 6 gelagert ist, so dass die vordere Trajektorie im Wesentlichen eine Form eines Kreisbogens hat. Dabei kann der Injektorkörper 6, insbesondere die erste Injektorkörperseitenwand 12, eine Injektorkörperaussparung 28 aufweisen, in der die Achse 23 beispielsweise drehbar gelagert ist. Die Schwenkvorrichtung 29 kann die Auflagewand 22 und die erste Seitenwand 26 sowie insbesondere die zweite Seitenwand 27 aufweisen. Die Schwenkvorrichtung 29 kann einen ersten Arm 24 aufweisen, der an der Achse 23 befestigt ist und die Auflagewand 22 und die erste Seitenwand 26 sowie insbesondere die zweite Seitenwand 27 bildet. Zudem kann die Schwenkvorrichtung 29 einen zweiten Arm 25 aufweisen, der an der Achse 23 befestigt ist und den Verlagerungsmechanismusanschlag 30 bildet. Der zweite Arm 25 kann beispielsweise in dem Innenraum 7 angeordnet sein, wie es in Figuren 1 bis 4 dargestellt ist. In diesem Fall kann der Injektorkörper 6 einen Schlitz aufweisen, via den der zweite Mitnehmer 83 sich von außerhalb des Injektorkörpers 6 in den Innenraum 7 erstreckt. Alternativ ist es denkbar, dass der zweite Arm 25 außerhalb des Injektorkörpers 6 angeordnet ist.

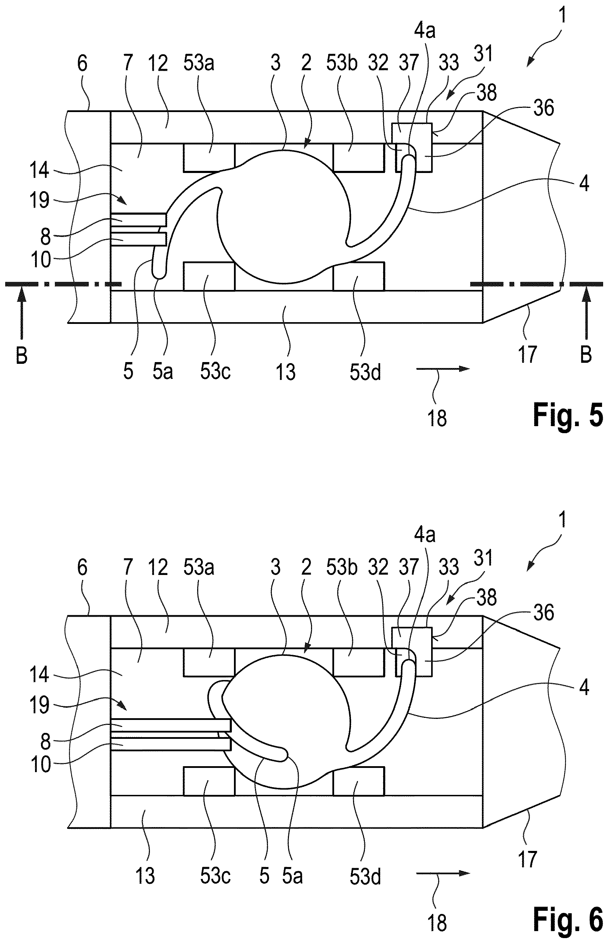

Figuren 5 bis 11 zeigen, dass der vordere Verlagerungsmechanismus 31 einen Mechanismusgleiter 33 aufweisen kann, der längsverlagerbar an dem Injektorkörper 6 gelagert ist, so dass die vordere Trajektorie im Wesentlichen gerade ist. Der Mechanismusgleiter 33 kann parallel zu der optischen Achse 3a längsverlagerbar sein, wodurch die vordere Trajektorie parallel zu der optischen Achse 3a orientiert ist. Um den Mechanismusgleiter 33 längsverlagerbar an dem Injektorkörper 6 zu lagern, kann der Injektorkörper 6, insbesondere die erste Injektorkörperseitenwand 12, eine Injektorkörperaussparung 38 aufweisen, in der der Mechanismusgleiter 33 angeordnet ist. Beispielsweise kann der Mechanismusgleiter 33 sich bis außerhalb des Injektorkörpers 6 erstrecken und dort eine Kontaktfläche 94 aufweisen, die von dem Verlagerungsmechanismusanschlag 95 gebildet ist und an die die der zweite Mitnehmer 93 anstößt, wenn der Griff 90 verlagert, vergleiche Figur 13. Figur 13 zeigt zudem, dass die Kontaktfläche 94 gegenüber der Einführrichtung 18 geneigt angeordnet sein kann.

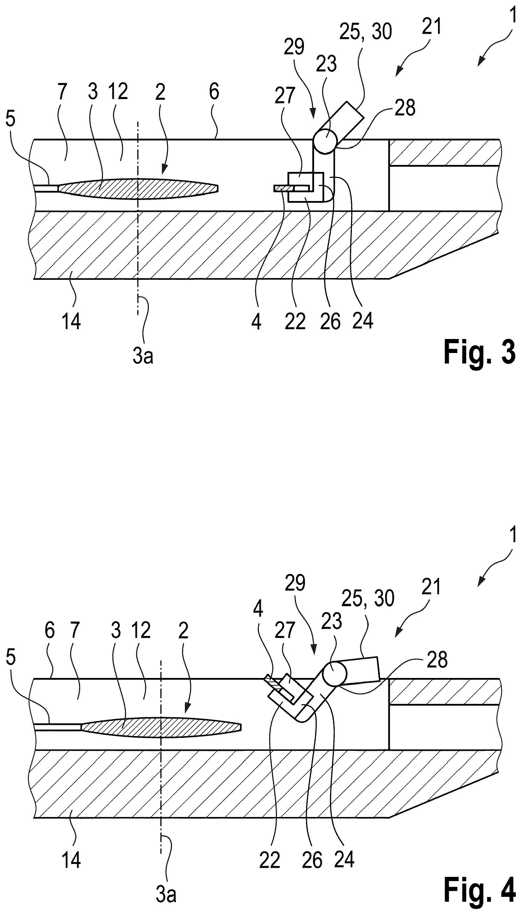

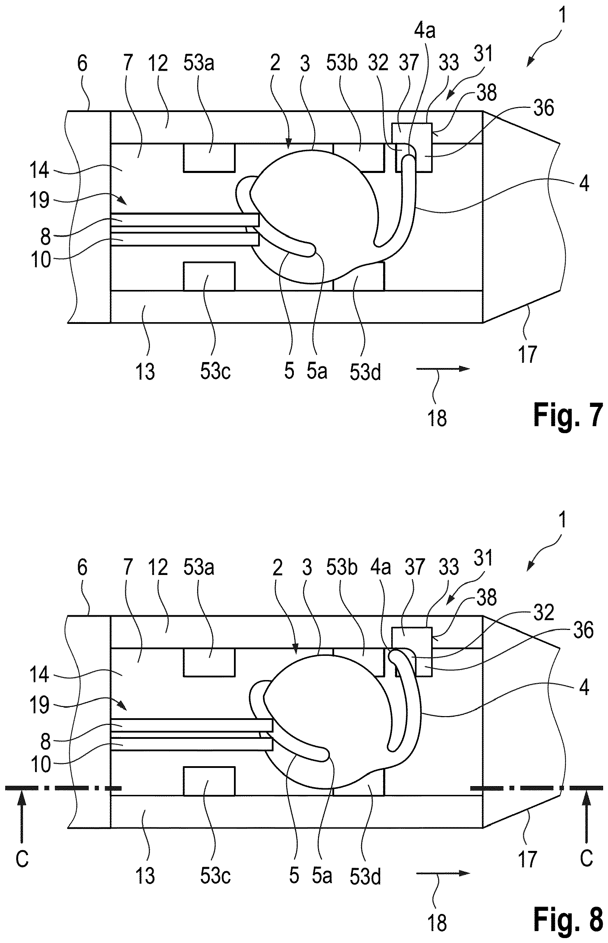

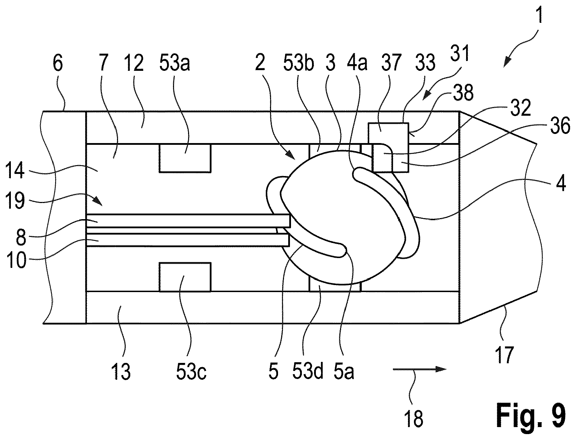

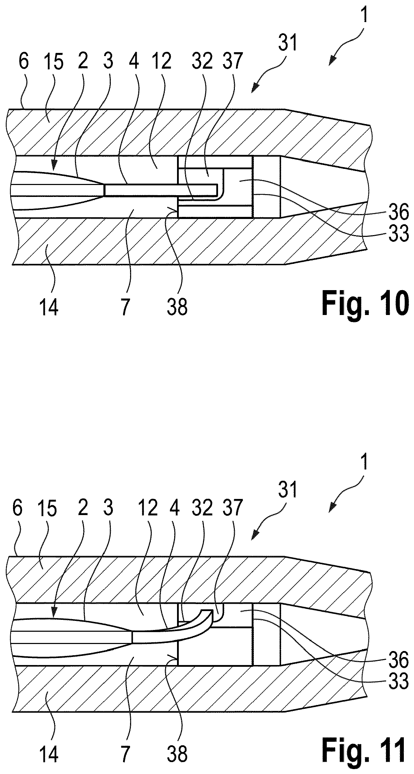

Figur 5 zeigt eine Draufsicht auf den Injektor 1 mit dem Mechanismusgleiter 33 zu einem ersten Zeitpunkt, Figur 6 zeigt die Draufsicht zu einem zweiten Zeitpunkt, Figur 7 zeigt die Draufsicht zu einem dritten Zeitpunkt, Figur 8 zeigt die Draufsicht zu einem vierten Zeitpunkt und Figur 9 zeigt die Draufsicht zu einem fünften Zeitpunkt, wobei in dieser Reihenfolge die Zeit weiter voranschreitet. Figur 10 zeigt einen Längsschnitt des Injektors 1 aus Figur 5 zu dem ersten Zeitpunkt und Figur 11 zeigt einen Längsschnitt des Injektors 1 aus Figur 8 zu dem vierten Zeitpunkt. Figur 3 zeigt einen zu dem Längsschnitt aus Figur 10 analogen Längsschnitt und Figur 4 zeigt einen zu dem Längsschnitt aus Figur 11 analogen Längsschnitt jeweils für die Ausführungsform des Injektors 1 mit der Schwenkvorrichtung 29. Zu dem ersten Zeitpunkt befindet sich die Intraokularlinse 2 in dem Lagerungszustand. Figuren 2, 3 und 10 zeigen, dass in dem Lagerungszustand die vordere Haptik 4 und die hintere Haptik 5 in einer Richtung senkrecht zu der optischen Achse 3a gesehen im Wesentlichen gerade angeordnet sein können. Insbesondere können die vordere Haptik 4 und die hintere Haptik 5 im Wesentlichen frei von einer mechanischen Spannung angeordnet sein. Indem die vordere Haptik 4 und die hintere Haptik 5 im Wesentlichen frei von der mechanischen Spannung sind, kann eine plastische Verformung der vorderen Haptik 4 und der hinteren Haptik 5 auch bei einer langen Zeitdauer einer Lagerung der Intraokularlinse 2 in dem Lagerungszustand vermieden werden.

Ausgehend von dem ersten Zeitpunkt (siehe Figuren 1 bis 3, 5 und 10) kann durch ein Verlagern des Griffs 80, 90, 96 der Kolben 19 in der Einführrichtung 18 verlagert werden. Der Kolben 19 verlagert zuerst das Längsende 5a der hinteren Haptik 5 auf den Optikkörper 3. Dies ist zu dem zweiten Zeitpunkt in Figur 6 dargestellt. Das Längsende 5a der hinteren Haptik 5 ist dabei in Richtung der optischen Achse 3a gesehen in dem Bereich des Optikkörpers 3 angeordnet. Zu dem zweiten Zeitpunkt kann der Optikkörper 3 noch in der gleichen Position wie zu dem ersten Zeitpunkt angeordnet sein, vergleiche Figuren 5 und 6.

Durch ein weiteres Verlagern des Griffs 80, 90, 96 und dadurch bedingtes Verlagern des Kolbens 19 wird der Optikkörper 3 zusammen mit dem auf dem Optikkörper 3 angeordneten Längsende 5a der hinteren Haptik 5 in der Einführrichtung 18 verlagert. Dies ist zu dem dritten Zeitpunkt in Figur 7 dargestellt. Dabei kann die vordere Haptik 4 in einem Bereich des Längsendes 4a der vorderen Haptik 4 an die erste Seitenwand 36 anstoßen, wodurch ein Verlagern der vorderen Haptik 4 begrenzt ist und sich das Längsende 4a der vorderen Haptik 4 dem Optikkörper 3 annähert.

Durch ein weiteres Verlagern des Griffs 80, 90, 96 nimmt der zweite Mitnehmer 83 den vorderen Verlagerungsmechanismus 21, 31 mit, so dass das Längsende 4a der vorderen Haptik 4 mit der vorderen Trajektorie verlagert. Dadurch vergrößert sich zu dem vierten Zeitpunkt eine Krümmung der vorderen Haptik 4 in einer Richtung senkrecht zu der optischen Achse gesehen, siehe Figuren 4 und 11. Der vordere Verlagerungsmechanismus 21, 31 kann eingerichtet sein, das Längsende 4a der vorderen Haptik 4 so weit entlang der vorderen Trajektorie zu verlagern (Figuren 4 und 11 zeigen das Ende der vorderen Trajektorie), dass bei einem Verlagern des Optikkörpers 3 in der Einführrichtung 18 das Längsende 4a der vorderen Haptik 4 auf den Optikkörper 3 verlagert. Dazu kann der vordere Verlagerungsmechanismus 21, 31 eingerichtet sein, die Auflagewand 22, 32 so weit entlang der vorderen Trajektorie zu verlagern, dass bei einem anschließenden Verlagern des Optikkörpers 3 in die Einführrichtung 18 der Optikkörper 3 auf eine dem Längsende 4a der vorderen Haptik 4 abgewandten Seite der Auflagewand 22, 32 gelangt. Es kann dementsprechend ein Zeitpunkt existieren, der zeitlich zwischen dem vierten Zeitpunkt und dem fünften Zeitpunkt liegt, in dem das Längsende 4a der vorderen Haptik 4 und der Optikkörper 3 auf einander abgewandten Seiten der Auflagewand 22, 32 angeordnet sind. Zu dem fünften Zeitpunkt in Figur 9 ist dargestellt, dass ausgehend von dem vierten Zeitpunkt durch ein weiteres Verlagern des Griffs 80, 90, 96 und damit des Optikkörpers 3 der Optikkörper 3 in der Einführrichtung 18 die erste Seitenwand 36 überholt hat und somit das Längsende 4a der vorderen Haptik 4 in Richtung der optischen Achse 3a gesehen in dem Bereich des Optikkörpers 3 angeordnet ist.

Der Zeitpunkt, zu dem das Längsende 4a der vorderen Haptik 4 anfängt, sich zu verlagern, kann durch ein Variieren des Abstands des zweiten Mitnehmers 83 zu dem ersten Mitnehmer 82 eingestellt werden. In Figuren 4 bis 9 und 11 ist dargestellt, dass der Zeitpunkt, zu dem das Längsende 4a der vorderen Haptik 4 anfängt, sich zu verlagern, zeitlich nach dem Zeitpunkt liegt, an dem der Optikkörper 3 anfängt sich zu verlagern. Es ist aber ebenso denkbar, dass der der Zeitpunkt, zu dem das Längsende 4a der vorderen Haptik 4 anfängt, sich zu verlagern, zeitgleich oder früher ist als der Zeitpunkt, an dem der Optikkörper 3 anfängt, sich zu verlagern.

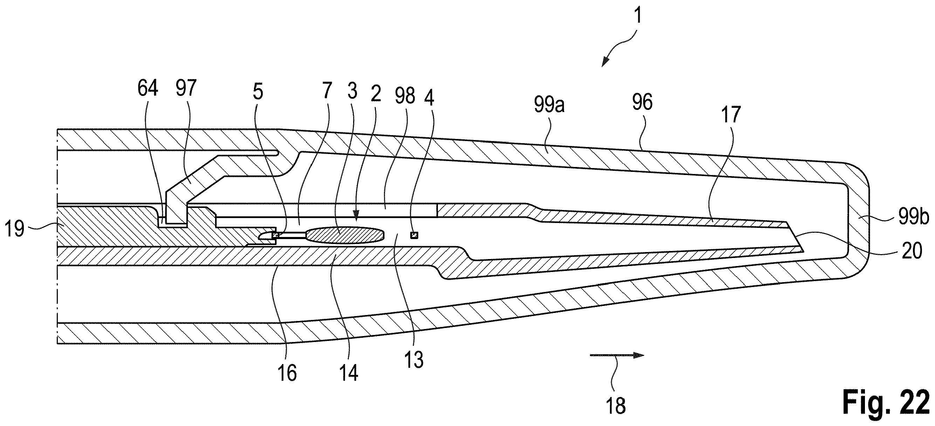

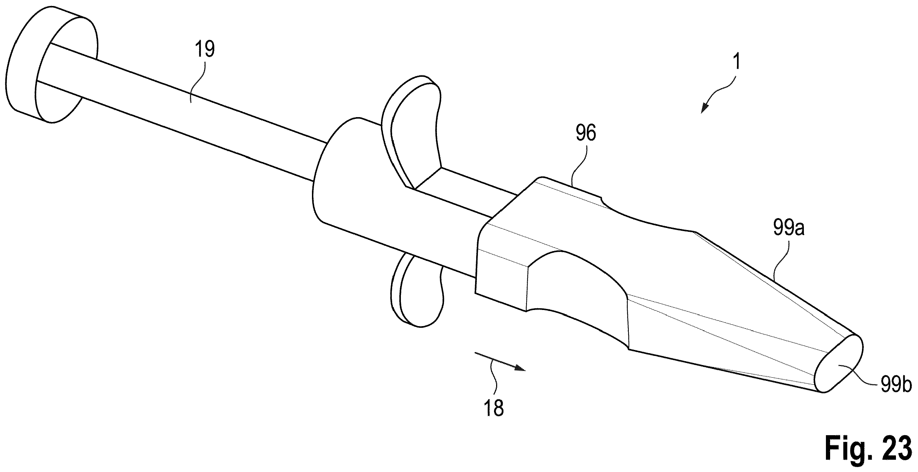

Figuren 12, 13, 22 und 23 zeigen, dass der Griff 80, 90, 96 eingerichtet sein kann, in der Einführrichtung 18 verlagert zu werden, um den Kolben 19 und den vorderen Verlagerungsmechanismus 21, 31 mitzunehmen. Zudem zeigen Figuren 12, 13, 22 und 23, dass der Griff 80, 90, 96 eine Außenhülle 81, 91, 99a aufweisen kann, die außerhalb des Injektorkörpers 6 angeordnet ist. Beispielsweise kann die Außenhülle 81, 91, 99a den Injektorkörper 6 in einer Umfangsrichtung bezüglich der Einführrichtung 18 vollständig umhüllen. Es ist auch denkbar, dass der Griff 80, 90, 96 in Form einer Kappe ausgebildet ist, vergleiche Figuren 22 und 23. Mit der Kappe ist es möglich, die Injektorspitze 17 in dem Lagerungszustand zu schützen, insbesondere vor einer Kontamination. Die Kappe kann eine Kappenseitenwand 99b aufweisen (siehe Figuren 22 und 23), die an der Außenhülle 81, 91, 99a befestigt ist und das in dem Inneren der Außenhülle 81, 91, 99a und von der Außenhülle 81, 91, 99a begrenzte Loch vollständig abdeckt. Die Kappenseitenwand 99b ist dabei in der Einführrichtung 18 beabstandet von der Öffnung 20 angeordnet.

Wie es aus Figuren 12, 13, 22 und 23 ersichtlich ist, kann der erste Mitnehmer 82, 92, 97 von einem Vorsprung gebildet sein, der von der Außenhülle 81, 91, 99a nach innen vorsteht. Der erste Mitnehmer 82, 92, 97 kann sich durch den Schlitz erstrecken, via den auch der zweite Mitnehmer 83, 93 sich von außerhalb des Injektorkörpers 6 in den Innenraum 7 erstreckt. Alternativ ist denkbar, dass der Injektorkörper 6 einen weiteren Schlitz 98 aufweist (siehe Figur 22), durch den der erste Mitnehmer 82, 92, 97 sich von außerhalb des Injektorkörpers 6 in den Innenraum 7 erstreckt. Um den ersten Mitnehmer 82, 92, 97 mit dem Kolben 19 zu koppeln, kann der Kolben 19 eine Mitnehmeraufnahme 64, siehe insbesondere Figur 22 in Form einer Aussparung aufweisen, in die der erste Mitnehmer 82, 92, 97 eingreift, wodurch der erste Mitnehmer 82, 92, 97 lösbar mit dem Kolben 19 gekoppelt ist.

Der zweite Mitnehmer 83, 93 kann von einem Vorsprung gebildet sein, der von der Außenhülle 81, 91, 99a nach innen vorsteht. Figur 12 zeigt, dass der Vorsprung eine erste Flanke 84, eine zweite Flanke 85 und eine dritte Flanke 86 aufweisen kann, die in dieser Reihenfolge unmittelbar aneinander angrenzen. Die erste Flanke 84 und die dritte Flanke 86 können im Wesentlichen parallel zu der Einführrichtung 18 angeordnet sein und die zweite Flanke 85 kann geneigt zu der Einführrichtung 18 angeordnet sein und somit den zweiten Mitnehmer 83 bilden.

Der Griff 80, 90, 96 kann eingerichtet sein, von dem verbliebenen Injektor 1 getrennt zu werden, wenn das Längsende 5a der hinteren Haptik 5 auf den Optikkörper 3 verlagert ist, das Längsende 4a der vorderen Haptik 4 entlang der vorderen Trajektorie verlagert ist und der Optikkörper 3 mittels des Kolbens 19 in der Einführrichtung 18 verlagert ist. Ein weiteres Verlagern der Intraokularlinse 2 zu der Öffnung 20 hin und via die Öffnung 20 aus dem Injektor 1 heraus kann durch ein weiteres Verlagern des Kolbens 19 erfolgen, beispielsweise indem der Kolben 19 in der Einführrichtung 18 gedrückt wird. Dazu kann der Kolben 19 sich entgegen der Einführrichtung 18 nach außerhalb des Injektorkörpers 6 erstrecken und eingerichtet sein, dort von einer Hand gedrückt zu werden. Alternativ ist denkbar, dass der Injektor 1 einen weiteren Kolben aufweist, der sich entgegen der Einführrichtung 18 nach außerhalb des Injektorkörpers 6 erstreckt. Der Kolben 19 und der weitere Kolben befinden sich entweder in einem Entkopplungszustand, in dem der Kolben 19 und der weitere Kolben relativ zueinander längsverlagerbar sind, oder in einem Kopplungszustand, in dem der Kolben 19 und der weitere Kolben starr in Einführrichtung 18 miteinander gekoppelt sind. Durch das Verlagern des Kolbens 19 mit dem Griff 80, 90, 96 können der Kolben 19 und der weitere Kolben in den Kopplungszustand gebracht werden. Dadurch kann der Injektor 1 insgesamt kürzer ausgebildet werden als in dem Fall, dass der weitere Kolben nicht vorgesehen ist.

Wie es aus Figuren 16 bis 18 und 20 ersichtlich ist, kann der hintere Verlagerungsmechanismus 100, 130, 160 eine Gleitfläche 117, 147, 177, 207 aufweisen, an der das Längsende 5a der hinteren Haptik 5 eingerichtet ist, entlang zu gleiten, wenn der Kolben 19 in der Einführrichtung 18 verlagert, und die so gegen die Einführrichtung 18 geneigt angeordnet ist, dass dabei die hintere Trajektorie eine hintere Komponente parallel zu der optischen Achse 3a hat. Dabei kann die hintere Komponente in der gleichen Richtung wie die vordere Komponente orientiert sein. Indem das Längsende 5a der hinteren Haptik 5 an der Gleitfläche 117, 147, 177, 207 entlang gleitet, vergrößert sich eine Krümmung der hinteren Haptik 5 in einer Richtung senkrecht zu der optischen Achse 3a gesehen. Nachdem das Längsende 5a der hinteren Haptik 5 entlang der Gleitfläche 117, 147, 177, 207 verlagert wurde, gelangt das Längsende 5a der hinteren Haptik 5 auf den Optikkörper 3, vergleiche Figur 6.

Figuren 1, 5 bis 9 und 14 bis 18 zeigen, dass der Kolben 19 einen Stößel 8, der eingerichtet ist, den Optikkörper 3 zu verlagern, und einen ersten Gleiter 10, 61, 101, 131, 161 aufweisen kann, der ein Teil des hinteren Verlagerungsmechanismus 100, 130, 160 ist und eingerichtet ist, die hintere Haptik 5 in einem Bereich des Längsendes 5a der hinteren Haptik 5 zu kontaktieren und somit auf den Optikkörper 3 zu verlagern. Der erste Gleiter 10, 61, 101, 131, 161 kann dabei in einer Querrichtung, die senkrecht zu der Einführrichtung 18 und senkrecht zu der optischen Achse 3a orientiert ist, zwischen dem Stößel 8 und der zweiten Injektorkörperseitenwand 13 angeordnet sein.

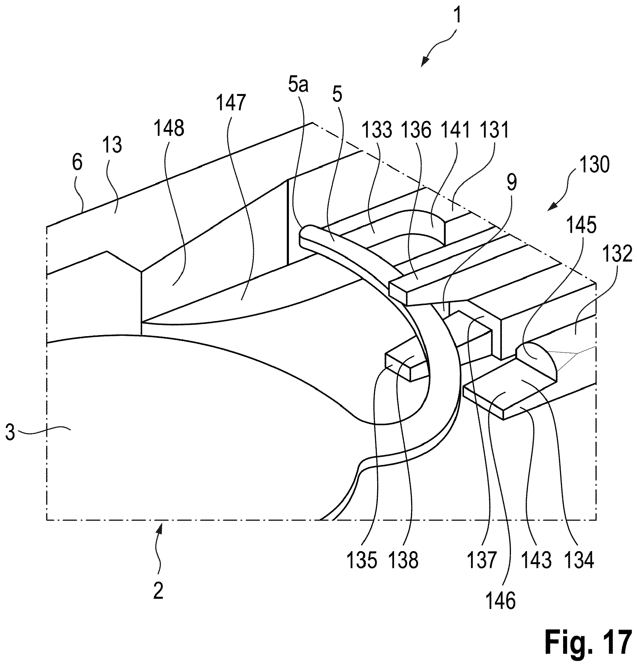

Der Injektorkörper 6 kann eine Seitenfläche 118, 148, 178, 208 aufweisen (siehe Figuren 16 bis 18 und 20), die den Innenraum 7 begrenzt, gegen die der erste Gleiter 10, 61, 101, 131, 161 vorgespannt ist, an der der erste Gleiter 10, 61, 101, 131, 161 entlang gleitet, wenn der Kolben 19 in der Einführrichtung 18 verlagert, und die so gegen die Einführrichtung 18 geneigt ist, dass mit zunehmender Verlagerung des Kolbens 19 in der Einführrichtung 18 der erste Gleiter 10, 61, 101, 131, 161 das Längsende 5a der hinteren Haptik 5 näher zu der optischen Achse 3a verlagert. Die Seitenfläche 118, 148, 178, 208 kann unmittelbar an die Gleitfläche 117, 147, 177, 207 angrenzen. Die Seitenfläche 148, 178, 208 kann beispielsweise in einer Querrichtung, die senkrecht zu der Einführrichtung 18 und senkrecht zu der optischen Achse 3a orientiert ist, außen von der Gleitfläche 147, 177, 207 angeordnet sein (siehe Figuren 17, 18 und 20) oder die Seitenfläche 118 kann beispielsweise in der Querrichtung außen von der Gleitfläche 117 angeordnet sein (siehe Figur 16). Der Kolben 19 kann ein Gelenk (nicht in den Figuren dargestellt), insbesondere ein Filmgelenk, aufweisen, mit dem der erste Gleiter 10, 61, 101, 131, 161 verschwenkbar an dem verbliebenen Kolben 19 befestigt ist. Damit kann der erste Gleiter 10, 61, 101, 131, wenn er an der Seitenfläche 118, 148, 178, 208 entlang gleitet, besonders einfach nach innen verlagern. Die Drehachse des Gelenks kann beispielsweise im Wesentlichen parallel zu der optischen Achse 3a sein.

Insbesondere Figuren 16 bis 18 zeigen, dass der Stößel 8 an seinem dem Optikkörper 3 zugewandten Ende eine Stößelausnehmung 9 aufweisen kann, die eingerichtet ist, die hintere Haptik 5 und den Optikkörper 3 aufzunehmen. Dabei kann der Stößel 8 einen ersten Zahn 105, 135, 165 des Stößels 8 aufweisen, wobei der erste Zahn 105, 135, 165 des Stößels 8 eine Ansteigeflanke 108, 138, 168 des Stößels 8 aufweist, an der die hintere Haptik 5 eingerichtet ist, entlang zu gleiten, wenn der Kolben 19 in der Einführrichtung 18 verlagert wird, und die so gegen die Einführrichtung 18 geneigt angeordnet ist, dass die hintere Haptik 5 im Bereich der Stößelausnehmung 9 parallel zu der optischen Achse 3a und in der gleichen Richtung wie die hintere Komponente verlagert. Zudem kann der Stößel 8 einen zweiten Zahn 106, 136, 166 des Stößels 8 aufweisen, wobei der zweite Zahn 106, 136, 166 des Stößels 8 die Stößelausnehmung 9 an einer der Ansteigeflanke 108, 138, 168 abgewandten Seite der Stößelausnehmung 9 begrenzt.

Der erste Gleiter 101, 131 kann eine Gleiterausnehmung 103, 133 des ersten Gleiters 101, 131 aufweisen, vergleiche Figuren 16 und 17. Gemäß Figur 16 kann der erste Gleiter 101 einen ersten Zahn 109 des ersten Gleiters 101 und einen zweiten Zahn 110 des ersten Gleiters 101 aufweisen, die die Gleiterausnehmung 103 des ersten Gleiters 101 in einer Richtung parallel zu der optischen Achse 3a an einander abgewandten Seiten begrenzen. Der erste Zahn 109 des ersten Gleiters 101 kann eingerichtet sein, die Seitenfläche 118 zu kontaktieren, siehe Figuren 16 und 17. Zudem kann der erste Zahn 109 des ersten Gleiters 101 eine Ansteigeflanke 112 des ersten Gleiters 101 aufweisen, an der die hintere Haptik 5 eingerichtet ist, entlang zu gleiten, wenn der Kolben 19 in der Einführrichtung 18 verlagert wird, und die so gegen die Einführrichtung 18 geneigt angeordnet ist, dass die hintere Haptik 5 im Bereich der Gleiterausnehmung 103 des ersten Gleiters 101 parallel zu der optischen Achse 3a und in der gleichen Richtung wie die hintere Komponente verlagert. Gemäß Figur 17 kann der erste Gleiter 131 einen ersten Zahn 139 des ersten Gleiters 131 aufweisen, der die Gleiterausnehmung 133 des ersten Gleiters 131 in einer Querrichtung begrenzt, die senkrecht zu der Einführrichtung 18 und senkrecht zu der optischen Achse 3a orientiert ist.

Figuren 16 bis 18 zeigen, dass der Kolben 19 einen zweiten Gleiter 102, 132, 162 aufweisen kann, der in einer Querrichtung, die senkrecht zu der Einführrichtung 18 und senkrecht zu der optischen Achse 3a orientiert ist, zwischen dem Stößel 8 und der ersten Injektorkörperseitenwand 12 angeordnet ist. Der zweite Gleiter 102, 132, 162 kann einen ersten Zahn 113, 143, 173 des zweiten Gleiters 102, 162 und einen zweiten Zahn 114, 174 des zweiten Gleiters 102, 162 aufweisen, die eine Gleiterausnehmung 104, 134, 164 des zweiten Gleiters 102, 162 in einer Richtung parallel zu der optischen Achse 3a an einander abgewandten Seiten begrenzen. Der erste Zahn 113, 143, 173 des zweiten Gleiters 102, 162 kann eine Ansteigeflanke 116, 146, 176 des zweiten Gleiters 102, 162 aufweisen, an der die hintere Haptik 5 eingerichtet ist, entlang zu gleiten, wenn der Kolben 19 in der Einführrichtung 18 verlagert wird, und die so gegen die Einführrichtung 18 geneigt angeordnet ist, dass die hintere Haptik 5 im Bereich der Gleiterausnehmung 104, 134, 164 des zweiten Gleiters 102, 162 parallel zu der optischen Achse 3a und in der gleichen Richtung wie die hintere Komponente verlagert.

In Figur 21 ist eine Ausführungsform für den zweiten Gleiter 222 abgebildet. Der zweite Gleiter 222 weist eine Ansteigefläche 236 des zweiten Gleiters 222, eine Optikkörperanschlagefläche 241, eine Haptikauflagefläche 243 und eine Stirnfläche 235 des zweiten Gleiters 222 auf, die in dieser Reihenfolge entgegen der Einführrichtung 18 nebeneinander angeordnet sind. An der Ansteigefläche 236 des zweiten Gleiters 222 ist die hintere Haptik 5 eingerichtet ist, entlang zu gleiten, wenn der Kolben 19 in der Einführrichtung 18 verlagert wird, und die so gegen die Einführrichtung 18 geneigt angeordnet ist, dass die hintere Haptik 5 im Bereich des zweiten Gleiters 222 parallel zu der optischen Achse 3a und in der gleichen Richtung wie die hintere Komponente verlagert. Die Normale der Optikkörperanschlagefläche 241 ist im Wesentlichen parallel zu der Einführrichtung 18 orientiert und ist dazu vorgesehen, dass bei einem Verlagern des Optikkörpers 3 der Optikkörper 3 daran anschlägt. Die Normale der Stirnfläche 235 des zweiten Gleiters 222 ist im Wesentlichen parallel zu der Einführrichtung 18 orientiert und ist dazu vorgesehen, dass bei einem Verlagern des Optikkörpers 3 die hintere Haptik 5 daran anschlägt. Die Haptikauflagefläche 243 ist im Wesentlichen senkrecht zu der Stirnfläche 235 des zweiten Gleiters 222 angeordnet.

Figuren 16 bis 18 zeigen, dass der Stößel 8 eine Stirnfläche 107, 137, 167 des Stößels 8 aufweisen kann, die die Stößelausnehmung 9 in der Einführrichtung 18 begrenzt. Der erste Gleiter 101, 131, 161 kann eine Stirnfläche 111, 141, 171 des ersten Gleiters 101, 131, 161 aufweisen, die die Gleiterausnehmung 103, 133, 163 des ersten Gleiters 101, 131, 161 in der Einführrichtung 18 begrenzt. Der zweite Gleiter 102, 132, 162 kann eine Stirnfläche 115, 145, 175 des zweiten Gleiters 102, 162 aufweisen, die die Gleiterausnehmung 104, 134, 164 des zweiten Gleiters 102, 162 in der Einführrichtung 18 begrenzt. Die Stirnfläche 107, 137, 167 des Stößels 8, die Stirnfläche 111, 141, 171 des ersten Gleiters 101, 131, 161 und optional die Stirnfläche 115, 145, 175 des zweiten Gleiters 102, 162 können dabei in unterschiedlichen Positionen in der Einführrichtung 18 angeordnet sein. Durch Wählen der Positionen in der Einführrichtung 18 kann eingestellt werden, welche Bereiche der hinteren Haptik 5 in welcher Reihenfolge zu dem Optikkörper 3 hin verlagert werden.

Figuren 18 und 19 zeigen eine Ausführungsform für den ersten Gleiter 161, bei der der erste Gleiter 161 in einer Richtung parallel zu der optischen Achse 3a und in der gleichen Richtung wie die vordere Komponente vorgespannt ist. In dem Geschlossenzustand des Deckels 15 ist der erste Gleiter 161 somit gegen den Deckel 15 vorgespannt. Der Deckel 15 weist einen Deckelvorsprung 181 auf, der eingerichtet ist, dass der erste Gleiter 161 an dem Deckelvorsprung 181 entlang gleitet, wenn der Kolben 19 in der Einführrichtung 18 verlagert. Der Deckelvorsprung 181 weist eine erste Vorsprungflanke 182 auf, an die der erste Gleiter 161 entlang gleitet und die so gegen die Einführrichtung 18 geneigt angeordnet ist, dass der erste Gleiter 161 in einer Richtung mit einer Komponente parallel zu der optischen Achse 3a und entgegen der vorderen Komponente verlagert. Der erste Gleiter 161 weist einen Zahn 169 auf, der eine Gleiterausnehmung 163 begrenzt, die eingerichtet ist, die hintere Haptik 5 aufzunehmen. Der Deckelvorsprung 181 weist eine dritte Vorsprungflanke 184 auf, die in der Einführrichtung 18 vor der ersten Vorsprungflanke 182 angeordnet ist und die so gegen die Einführrichtung 18 geneigt angeordnet ist, dass der erste Gleiter 161 in einer Richtung mit einer Komponente parallel zu der optischen Achse 3a und in der gleichen Richtung wie die vordere Komponente verlagert. Der Zahn 169 ist dabei eingerichtet, die hintere Haptik 5 in der Richtung mit der Komponente parallel zu der optischen Achse 3a und in der gleichen Richtung wie die vordere Komponente zu verlagern. Zudem kann der Deckelvorsprung 181 eine zweite Vorsprungflanke 183 aufweisen, die in der Einführrichtung 18 zwischen der ersten Vorsprungflanke 182 und der dritten Vorsprungflanke 184 angeordnet ist. Der erste Gleiter 161 kann zudem einen Gleitervorsprung 186 aufweisen, der in Richtung zu dem Deckel 15 in dessen Geschlossenzustand hin von dem verbliebenen ersten Gleiter 161 vorsteht. Der Deckel 15 kann eines von einem Zentrierstift 179 und einer Zentrieraussparung 180 aufweisen und die erste Injektorkörperseitenwand 12 oder die zweite Injektorkörperseitenwand 13 kann das andere von dem Zentrierstift 179 und der Zentrieraussparung 180 aufweisen, wobei in dem Geschlossenzustand der Zentrierstift in die Zentrieraussparung eingreift. Dadurch kann der Deckelvorsprung 181 mit einer hohen Genauigkeit relativ zu dem ersten Gleiter 161 angeordnet werden.

Wie es aus Figur 20 ersichtlich ist, kann der Deckel 15 eine Deckelführung 209 aufweisen, die in der Einführrichtung 18 von der Seitenfläche 208 angeordnet ist und die eingerichtet ist, dass der erste Gleiter 101, 131, 161 an der Deckelführung 209 entlang gleitet. Durch das Vorsehen der Deckelführung 209 kann das Längsende 5a der hinteren Haptik 5 näher zu der optischen Achse 3a verlagert werden als in dem Fall, dass die Deckelführung 209 nicht vorgesehen ist.

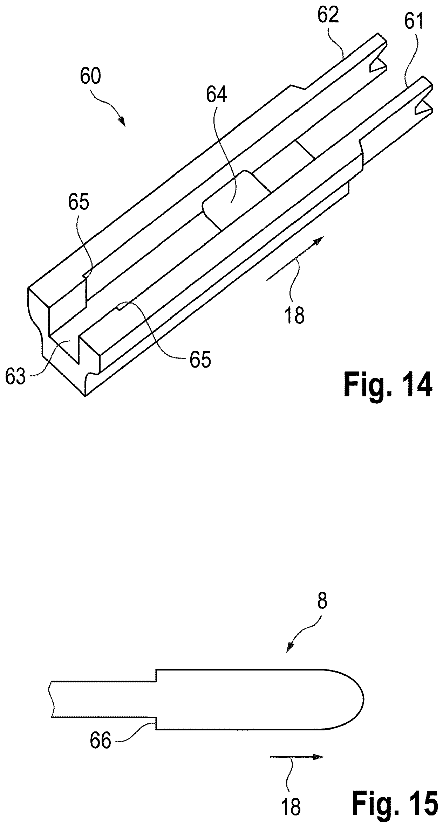

Figur 14 zeigt, dass der Kolben 19 eine Gleiteranordnung 60 aufweisen kann, die den ersten Gleiter 61 und eine Stößelaufnahme 63 aufweisen kann, in der der Stößel 8 längsverlagerbar relativ zu der Gleiteranordnung 60 angeordnet ist, wobei die Gleiteranordnung 60 mit dem ersten Mitnehmer 82, 92, 97 gekoppelt ist, wodurch der erste Mitnehmer 82, 92, 97 mit dem Kolben 19 gekoppelt ist, und einen Anschlag 65 aufweist, wobei der Stößel 8 eine Stößelverdickung 66 aufweist (siehe Figur 15), die eingerichtet ist, gegen den Anschlag 65 anzuschlagen, wenn der Stößel 8 entgegen der Einführrichtung 18 relativ zu der Gleiteranordnung 60 verlagert. Die Gleiteranordnung 60 kann eine Mitnehmeraufnahme 64 aufweisen, in die der erste Mitnehmer 82, 92, 97 eingreift, um den ersten Mitnehmer 82, 92, 97 mit der Gleiteranordnung 60 zu koppeln, wodurch der erste Mitnehmer 82, 92, 97 lösbar mit dem Kolben 19 gekoppelt ist. Indem der erste Mitnehmer 82, 92, 97 aus der Mitnehmeraufnahme 65 gelangt, kann der erste Mitnehmer 82, 92, 97 von dem Kolben 19 entkoppelt werden. Dabei ist auch denkbar, dass dadurch der Griff 80, 90, 96 von dem verbliebenen Injektor 1 getrennt werden kann.

### Bezugszeichenliste

- 1: Injektor
- 2: Intraokularlinse
- 3: Optikkörper
- 3a: optische Achse
- 4: vordere Haptik
- 4a: Längsende der vorderen Haptik
- 5: hintere Haptik
- 5a: Längsende der hinteren Haptik
- 6: Injektorkörper
- 7: Innenraum
- 8: Stößel
- 9: Stößelausnehmung
- 10: erster Gleiter
- 12: erste Injektorkörperseitenwand
- 13: zweite Injektorkörperseitenwand
- 14: Injektorkörperboden
- 15: Deckel
- 16: Deckelgelenk
- 17: Injektorspitze
- 18: Einführrichtung
- 19: Kolben
- 20: Öffnung
- 21: vorderer Verlagerungsmechanismus
- 22: Auflagewand
- 23: Achse
- 24: erster Arm
- 25: zweiter Arm
- 26: erste Seitenwand
- 27: zweite Seitenwand
- 28: Injektorkörperaussparung
- 29: Schwenkvorrichtung
- 30: Verlagerungsmechanismusanschlag

- 31: vorderer Verlagerungsmechanismus
- 32: Auflagewand
- 33: Mechanismusgleiter
- 36: erste Seitenwand
- 37: zweite Seitenwand
- 38: Injektorkörperaussparung

- 51: erstes Podest
- 52: zweites Podest
- 53a: erster Haltevorsprung
- 53b: zweiter Haltevorsprung
- 53c: dritter Haltevorsprung
- 53d: vierter Haltevorsprung

- 60: Gleiteranordnung
- 61: erster Gleiter
- 62: zweiter Gleiter
- 63: Stößelaufnahme
- 64: Mitnehmeraufnahme
- 65: Anschlag
- 66: Stößelverdickung

- 80: Griff
- 81: Außenhülle
- 82: erster Mitnehmer
- 83: zweiter Mitnehmer
- 84: erste Flanke
- 85: zweite Flanke
- 86: dritte Flanke

- 90: Griff
- 91: Außenhülle
- 92: erster Mitnehmer
- 93: zweiter Mitnehmer
- 94: Kontaktfläche
- 95: Verlagerungsmechanismusanschlag
- 96: Griff
- 97: zweiter Mitnehmer
- 98: Schlitz

- 100: hinterer Verlagerungsmechanismus
- 101: erster Gleiter
- 102: zweiter Gleiter
- 103: Gleiterausnehmung des ersten Gleiters
- 104: Gleiterausnehmung des zweiten Gleiters
- 105: erster Zahn des Stößels
- 106: zweiter Zahn des Stößels
- 107: Stirnfläche des Stößels
- 108: Ansteigeflanke des Stößels
- 109: erster Zahn des ersten Gleiters
- 110: zweiter Zahn des ersten Gleiters
- 111: Stirnfläche des ersten Gleiters
- 112: Ansteigeflanke des ersten Gleiters
- 113: erster Zahn des zweiten Gleiters
- 114: zweiter Zahn des zweiten Gleiters
- 115: Stirnfläche des zweiten Gleiters
- 116: Ansteigeflanke des zweiten Gleiters
- 117: Gleitfläche
- 118: Seitenfläche

- 130: hinterer Verlagerungsmechanismus
- 131: erster Gleiter
- 132: zweiter Gleiter
- 133: Gleiterausnehmung des ersten Gleiters
- 134: Gleiterausnehmung des zweiten Gleiters
- 135: erster Zahn des Stößels
- 136: zweiter Zahn des Stößels
- 137: Stirnfläche des Stößels
- 138: Ansteigeflanke des Stößels
- 139: erster Zahn des ersten Gleiters
- 141: Stirnfläche des ersten Gleiters
- 143: erster Zahn des zweiten Gleiters
- 145: Stirnfläche des zweiten Gleiters
- 146: Ansteigeflanke des zweiten Gleiters
- 147: Gleitfläche
- 148: Seitenfläche

- 160: hinterer Verlagerungsmechanismus
- 161: erster Gleiter
- 162: zweiter Gleiter
- 163: Gleiterausnehmung des ersten Gleiters
- 164: Gleiterausnehmung des zweiten Gleiters
- 165: erster Zahn des Stößels
- 166: zweiter Zahn des Stößels
- 167: Stirnfläche des Stößels
- 168: Ansteigeflanke des Stößels
- 169: Zahn des ersten Gleiters
- 171: Stirnfläche des ersten Gleiters
- 173: erster Zahn des zweiten Gleiters
- 174: zweiter Zahn des zweiten Gleiters
- 175: Stirnfläche des zweiten Gleiters
- 176: Ansteigeflanke des zweiten Gleiters
- 177: Gleitfläche
- 178: Seitenfläche
- 179: Zentrierstift
- 180: Zentrieraussparung
- 181: Deckelvorsprung
- 182: erste Vorsprungsflanke
- 183: zweite Vorsprungsflanke
- 184: dritte Vorsprungsflanke
- 186: Gleitervorsprung

- 207: Gleitfläche
- 208: Seitenfläche
- 209: Deckelführung

- 222: zweiter Gleiter
- 235: Stirnfläche des zweiten Gleiters
- 236: Ansteigefläche des zweiten Gleiters
- 241: Optikkörperanschlagefläche
- 243: Haptikauflagefläche

## Patentansprüche

1. Injektor für ein Einführen einer Intraokularlinse (2) in den Kapselsack eines Auges, mit einem Injektorkörper (6), der einen Innenraum (7) begrenzt, in dem die Intraokularlinse (2) in einem Lagerungszustand der Intraokularlinse (2) anzuordnen ist, einer Injektorspitze (17), die eine Öffnung (20) aufweist, einem Kolben (19), der eingerichtet ist, die Intraokularlinse (2) durch ein Längsverlagern des Kolbens (19) in einer Einführrichtung (18) hin zu der Öffnung (20) via die Öffnung (20) aus dem Injektor (1) heraus zu verlagern, wobei die Intraokularlinse (2) einen Optikkörper (3) mit einer optischen Achse (3a) und eine auf die Einführrichtung (18) bezogene hintere Haptik (5) und eine auf die Einführrichtung (18) bezogene vordere Haptik (4) aufweist, einem hinteren Verlagerungsmechanismus (100, 130, 160), der eingerichtet ist, durch ein Verlagern des Kolbens (19) ein dem Optikkörper (3) abgewandtes Längsende (5a) der hinteren Haptik (5) entlang einer hinteren Trajektorie auf den Optikkörper (3) zu verlagern, einem vorderen Verlagerungsmechanismus (21, 31), der eingerichtet ist, ein dem Optikkörper (3) abgewandtes Längsende (4a) der vorderen Haptik (4) entlang einer vorderen Trajektorie zu verlagern, die eine vordere Komponente hat, die parallel zu der optischen Achse (3a) ist, und einem Griff (80, 90, 96), der eingerichtet ist, von außerhalb des Injektors (1) bedienbar zu sein, sowie einen ersten Mitnehmer (82, 92, 97), der mit dem Kolben (19) gekoppelt ist und eingerichtet ist, durch ein Längsverlagern des Griffs (80, 90, 96) den Kolben (19) in der Einführrichtung (18) mitzunehmen, und einen zweiten Mitnehmer (83, 93) aufweist, der eingerichtet ist, den vorderen Verlagerungsmechanismus (21, 31) mitzunehmen, so dass durch das Längsverlagern des Griffs (80, 90, 96) das Längsende (5a) der hinteren Haptik (5) auf den Optikkörper (3) verlagerbar ist, das Längsende (4a) der vorderen Haptik (4) entlang der vorderen Trajektorie verlagerbar ist und der Optikkörper (3) mittels des Kolbens (19) in der Einführrichtung (18) verlagerbar ist.

2. Injektor gemäß Anspruch 1, wobei der vordere Verlagerungsmechanismus (21, 31) eine Auflagewand (22, 32), auf der in dem Lagerungszustand das Längsende (4a) der vorderen Haptik (4) aufliegt und die das Längsende (4a) der vorderen Haptik (4) in der Richtung der vorderen Trajektorie abstützt, und eine erste Seitenwand (26, 36) aufweist, die eingerichtet ist, dass die vordere Haptik (4) an die erste Seitenwand (26, 36) anstößt, wenn die vordere Haptik (4) in der Einführrichtung (18) verlagert.

3. Injektor gemäß Anspruch 1 oder 2, wobei der vordere Verlagerungsmechanismus (21) eine Schwenkvorrichtung (29) aufweist, die eine Achse (23) aufweist, die drehbar an dem Injektorkörper (6) gelagert ist, so dass die vordere Trajektorie im Wesentlichen eine Form eines Kreisbogens hat oder wobei der vordere Verlagerungsmechanismus (31) einen Mechanismusgleiter (33) aufweist, der längsverlagerbar an dem Injektorkörper (6) gelagert ist, so dass die vordere Trajektorie im Wesentlichen gerade ist, insbesondere ist die vordere Trajektorie parallel zu der optischen Achse (3a) orientiert.

4. Injektor gemäß einem der Ansprüche 1 bis 3, wobei der hintere Verlagerungsmechanismus (100, 130, 160) eine Gleitfläche (117, 147, 177, 207) aufweist, an der das Längsende (5a) der hinteren Haptik (5) eingerichtet ist, entlang zu gleiten, wenn der Kolben (19) in der Einführrichtung (18) verlagert, und die so gegen die Einführrichtung (18) geneigt angeordnet ist, dass dabei die hintere Trajektorie eine hintere Komponente parallel zu der optischen Achse (3a) hat, insbesondere ist die hintere Komponente in der gleichen Richtung wie die vordere Komponente orientiert.

5. Injektor gemäß einem der Ansprüche 1 bis 4, wobei der Kolben (19) einen Stößel (8), der eingerichtet ist, den Optikkörper (3) zu verlagern, und einen ersten Gleiter (10, 61, 101, 131, 161) aufweist, der ein Teil des hinteren Verlagerungsmechanismus (100, 130, 160) ist und eingerichtet ist, die hintere Haptik (5) in einem Bereich des Längsendes (5a) der hinteren Haptik (5) zu kontaktieren und somit auf den Optikkörper (3) zu verlagern.

6. Injektor gemäß Anspruch 5, wobei der Injektorkörper (6) eine Seitenfläche (118, 148, 178, 208) aufweist, die den Innenraum (7) begrenzt, gegen die der erste Gleiter (10, 61, 101, 131, 161) vorgespannt ist, an der der erste Gleiter (10, 61, 101, 131, 161) entlang gleitet, wenn der Kolben (19) in der Einführrichtung (18) verlagert, und die so gegen die Einführrichtung (18) geneigt ist, dass mit zunehmender Verlagerung des Kolbens (19) in der Einführrichtung (18) der erste Gleiter (10, 61, 101, 131, 161) das Längsende (5a) der hinteren Haptik (5) näher zu der optischen Achse (3a) verlagert.

7. Injektor gemäß Anspruch 5 oder 6, wobei der Stößel (8) an seinem dem Optikkörper (3) zugewandten Ende eine Stößelausnehmung (9) aufweist, die eingerichtet ist, die hintere Haptik (5) und den Optikkörper (3) aufzunehmen.

8. Injektor gemäß Anspruch 7, wobei der Stößel (8) einen ersten Zahn (105, 135, 165) des Stößels (8) aufweist, wobei der erste Zahn (105, 135, 165) des Stößels (8) eine Ansteigeflanke (108, 138, 168) des Stößels (8) aufweist, an der die hintere Haptik (5) eingerichtet ist, entlang zu gleiten, wenn der Kolben (19) in der Einführrichtung (18) verlagert wird, und die so gegen die Einführrichtung (18) geneigt angeordnet ist, dass die hintere Haptik (5) im Bereich der Stößelausnehmung (9) parallel zu der optischen Achse (3a) und in der gleichen Richtung wie die hintere Komponente verlagert.

9. Injektor gemäß einem der Ansprüche 5 bis 8, wobei der Kolben (19) eine Gleiteranordnung (60) aufweist, die den ersten Gleiter (10, 61, 101, 131, 161) und eine Stößelaufnahme (63) aufweist, in der der Stößel (8) längsverlagerbar relativ zu der Gleiteranordnung (60) angeordnet ist, wobei die Gleiteranordnung (60) mit dem ersten Mitnehmer (82, 92, 97) gekoppelt ist, wodurch der erste Mitnehmer (82, 92, 97) mit dem Kolben (19) gekoppelt ist, und einen Anschlag (65) aufweist, der Stößel (8) eine Stößelverdickung (66) aufweist, die eingerichtet ist, gegen den Anschlag (65) anzuschlagen, wenn der Stößel (8) entgegen der Einführrichtung (18) relativ zu der Gleiteranordnung (60) verlagert.

10. Injektor gemäß einem der Ansprüche 1 bis 9, wobei der erste Mitnehmer (82, 92, 97) lösbar mit dem Kolben (19) gekoppelt ist.

## Claims

1. Injector for inserting an intraocular lens (2) into the capsular bag of an eye, having an injector body (6) which delimits an interior (7) in which the intraocular lens (2) should be arranged in a storage state of the intraocular lens (2); an injector tip (17) which has an opening (20); a plunger (19) configured to displace the intraocular lens (2) out of the injector (1) via the opening (20) by way of a longitudinal displacement of the plunger (19) in an insertion direction (18) toward the opening (20), with the intraocular lens (2) having an optic body (3) with an optical axis (3a) and a rear haptic (5) in relation to the insertion direction (18) and a front haptic (4) in relation to the insertion direction (18); a rear displacement mechanism (100, 130, 160) configured to displace an optic body (3) distant longitudinal end (5a) of the rear haptic (5) onto the optic body (3) along a rear trajectory by way of a displacement of the plunger (19); a front displacement mechanism (21, 31) configured to displace an optic body (3) distant longitudinal end (4a) of the front haptic (4) along a front trajectory which has a front component parallel to the optical axis (3a); and a handle (80, 90, 96) which is configured to be operable from outside of the injector (1) and which has a first catch (82, 92, 97) which is coupled to the plunger (19) and is configured to carry along the plunger (19) in the insertion direction (18) by way of a longitudinal displacement of the handle (80, 90, 96) and has a second catch (83, 93) configured to carry along the front displacement mechanism (21, 31) so that, by way of the longitudinal displacement of the handle (80, 90, 96), the longitudinal end (5a) of the rear haptic (5) is displaceable onto the optic body (3), the longitudinal end (4a) of the front haptic (4) is displaceable along the front trajectory, and the optic body (3) is displaceable in the insertion direction (18) by means of the plunger (19).

2. Injector according to Claim 1, wherein the front displacement mechanism (21, 31) has a bearing wall (22, 32), which bears the longitudinal end (4a) of the front haptic (4) in the storage state and which supports the longitudinal end (4a) of the front haptic (4) in the direction of the front trajectory, and a first side wall (26, 36) configured so that the front haptic (4) abuts against the first side wall (26, 36) when the front haptic (4) is displaced in the insertion direction (18).

3. Injector according to Claim 1 or 2, wherein the front displacement mechanism (21) has a pivoting device (29) having a shaft (23) rotatably mounted on the injector body (6) such that the front trajectory substantially has the form of a circular arc, or wherein the front displacement mechanism (31) has a mechanism slider (33) mounted on the injector body (6) so as to be longitudinally displaceable such that the front trajectory is substantially straight; in particular, the front trajectory is oriented parallel to the optical axis (3a) .

4. Injector according to any one of Claims 1 to 3, wherein the rear displacement mechanism (100, 130, 160) has a sliding surface (117, 147, 177, 207) along which the longitudinal end (5a) of the rear haptic (5) is configured to slide when the plunger (19) is displaced in the insertion direction (18) and which is inclined counter to the insertion direction (18) so that the rear trajectory has in the process a rear component parallel to the optical axis (3a); in particular, the rear component is oriented in the same direction as the front component.

5. Injector according to any one of Claims 1 to 4, wherein the plunger (19) has a ram (8) which is configured to displace the optic body (3), and a first slider (10, 61, 101, 131, 161) which is a part of the rear displacement mechanism (100, 130, 160) and is configured to contact the rear haptic (5) in a region of the longitudinal end (5a) of the rear haptic (5) and consequently to displace the said rear haptic onto the optic body (3).

6. Injector according to Claim 5, wherein the injector body (6) has a side face (118, 148, 178, 208) which delimits the interior (7), against which the first slider (10, 61, 101, 131, 161) is prestressed, along which the first slider (10, 61, 101, 131, 161) slides when the plunger (19) is displaced in the insertion direction (18), and which is inclined counter to the insertion direction (18) so that, as the plunger (19) is displaced further in the insertion direction (18), the first slider (10, 61, 101, 131, 161) displaces the longitudinal end (5a) of the rear haptic (5) closer to the optical axis (3a) .

7. Injector according to Claim 5 or 6, wherein the ram (8) has a ram cutout (9) at its end facing the optic body (3), the said ram cutout being configured to accommodate the rear haptic (5) and the optic body (3).

8. Injector according to Claim 7, wherein the ram (8) has a first tooth (105, 135, 165) of the ram (8), with the first tooth (105, 135, 165) of the ram (8) having a rising flank (108, 138, 168) of the ram (8), on which the rear haptic (5) is configured to slide along when the plunger (19) is displaced in the insertion direction (18) and which is inclined counter to the insertion direction (18) so that the rear haptic (5) is displaced parallel to the optical axis (3a) in the region of the ram cutout (9) and is displaced in the same direction as the rear component.

9. Injector according to any one of Claims 5 to 8, wherein the plunger (19) has a slider arrangement (60) having the first slider (10, 61, 101, 131, 161) and a ram receptacle (63) in which the ram (8) is arranged so as to be longitudinally displaceable relative to the slider arrangement (60), the slider arrangement (60) being coupled to the first catch (82, 92, 97), as a result of which the first catch (82, 92, 97) is coupled to the plunger (19), and has a stop (65), the ram (8) having a ram thickening (66) configured to abut against the stop (65) when the ram (8) is displaced relative to the slider arrangement (60) counter to the insertion direction (18).

10. Injector according to any one of Claims 1 to 9, wherein the first catch (82, 92, 97) is releasably coupled to the plunger (19).

## Revendications

1. Injecteur permettant d'introduire une lentille intraoculaire (2) dans le sac capsulaire d'un oeil, comprenant un corps d'injecteur (6) qui délimite un espace intérieur (7) dans lequel la lentille intraoculaire (2) est à disposer dans un état de conservation de la lentille intraoculaire (2), une pointe d'injecteur (17) qui présente une ouverture (20), un piston (19) qui est conçu pour faire sortir la lentille intraoculaire (2) de l'injecteur (1) par l'intermédiaire de l'ouverture (20) par un décalage longitudinal du piston (19) dans une direction d'introduction (18) en direction de l'ouverture (20), dans lequel la lentille intraoculaire (2) présente un corps optique (3) pourvu d'un axe optique (3a) et une haptique arrière (5) par rapport à la direction d'introduction (18) et une haptique avant (4) par rapport à la direction d'introduction (18), un mécanisme de décalage arrière (100, 130, 160) qui est conçu pour décaler par un décalage du piston (19) une extrémité longitudinale (5a) détournée du corps optique (3) de l'haptique arrière (5) le long d'une trajectoire arrière en direction du corps optique (3), un mécanisme de décalage avant (21, 31) qui est conçu pour décaler une extrémité longitudinale (4a) détournée du corps optique (3) de l'haptique avant (4) le long d'une trajectoire avant qui présente une composante avant qui est parallèle à l'axe optique (3a), et une poignée (80, 90, 96) qui est conçue afin de pouvoir être commandée depuis l'extérieur de l'injecteur (1), ainsi qu'un premier entraîneur (82, 92, 97) qui est couplé au piston (19) et est conçu pour entraîner par un décalage longitudinal de la poignée (80, 90, 96) le piston (19) dans la direction d'introduction (18), et un présente un deuxième entraîneur (83, 93) qui est conçu pour entraîner le mécanisme de décalage avant (21, 31) de sorte que le décalage longitudinal de la poignée (80, 90, 96) permet de décaler l'extrémité longitudinale (5a) de l'haptique arrière (5) vers le corps optique (3), de décaler l'extrémité longitudinale (4a) de l'haptique avant (4) le long de la trajectoire avant, et de décaler le corps optique (3) au moyen du piston (19) dans la direction d'introduction (18).

2. Injecteur selon la revendication 1, dans lequel le mécanisme de décalage avant (21, 31) présente une paroi d'appui (22, 32) sur laquelle repose l'extrémité longitudinale (4a) de l'haptique avant (4) à l'état de conservation, et qui soutient l'extrémité longitudinale (4a) de l'haptique avant (4) dans la direction de la trajectoire avant, et présente une première paroi latérale (26, 36) qui est conçue de sorte que l'haptique avant (4) vient en butée contre la première paroi latérale (26, 36) lorsque l'haptique avant (4) est décalée dans la direction d'introduction (18).

3. Injecteur selon la revendication 1 ou 2, dans lequel le mécanisme de décalage avant (21) présente un dispositif pivotant (29) qui présente un axe (23) qui est monté rotatif au niveau du corps d'injecteur (6) de sorte que la trajectoire avant présente substantiellement une forme d'un arc de cercle, ou dans lequel le mécanisme de décalage avant (31) présente un coulisseau de mécanisme (33) qui est monté en décalage longitudinal au niveau du corps d'injecteur (6) de sorte que la trajectoire avant est substantiellement droite, en particulier que la trajectoire avant est orientée en parallèle à l'axe optique (3a).

4. Injecteur selon l'une quelconque des revendications 1 à 3, dans lequel le mécanisme de décalage arrière (100, 130, 160) présente une surface de glissement (117, 147, 177, 207), l'extrémité longitudinale (5a) de l'haptique arrière (5) étant conçue pour coulisser le long de celle-ci lorsque le piston (19) se décale dans la direction d'introduction (18), et qui est disposée en étant inclinée par rapport à la direction d'introduction (18) de telle sorte que la trajectoire arrière présente alors une composante arrière parallèle à l'axe optique (3a), en particulier que la composante arrière est orientée dans la même direction que la composante avant.

5. Injecteur selon l'une quelconque des revendications 1 à 4, dans lequel le piston (19) présente un poussoir (8) qui est conçu pour décaler le corps optique (3), et un premier coulisseau (10, 61, 101, 131, 161) qui fait partie du mécanisme de décalage arrière (100, 130, 160) et est conçu pour entrer en contact avec l'haptique arrière (5) dans une zone de l'extrémité longitudinale (5a) de l'haptique arrière (5) et pour la décaler ainsi vers le corps optique (3).

6. Injecteur selon la revendication 5, dans lequel le corps d'injecteur (6) présente une surface latérale (118, 148, 178, 208) qui délimite l'espace intérieur (7) contre lequel le premier coulisseau (10, 61, 101, 131, 161) est précontraint, le long de laquelle glisse le premier coulisseau (10, 61, 101, 131, 161) lorsque le piston (19) se décale dans la direction d'introduction (18), et qui est inclinée par rapport à la direction d'introduction (18) de telle sorte qu'avec un décalage croissant du piston (19) dans la direction d'introduction (18), le premier coulisseau (10, 61, 101, 131, 161) rapproche l'extrémité longitudinale (5a) de l'haptique arrière (5) de l'axe optique (3a).

7. Injecteur selon la revendication 5 ou 6, dans lequel le poussoir (8) présente à son extrémité tournée vers le corps optique (3) un évidement de poussoir (9) qui est conçu pour recevoir l'haptique arrière (5) et le corps optique (3).

8. Injecteur selon la revendication 7, dans lequel le poussoir (8) présente une première dent (105, 135, 165) du poussoir (8), dans lequel la première dent (105, 135, 165) du poussoir (8) présente un flanc montant (108, 138, 168) du poussoir (8), l'haptique arrière (5) étant conçue pour glisser le long de celui-ci lorsque le piston (19) est décalé dans la direction d'introduction (18), et qui est disposé en étant incliné par rapport à la direction d'introduction (18) de telle sorte que l'haptique arrière (5) se décale dans la zone de l'évidement de poussoir (9) en parallèle à l'axe optique (3a) et dans la même direction que la composante arrière.

9. Injecteur selon l'une quelconque des revendications 5 à 8, dans lequel le piston (19) présente un agencement de coulisseau (60) qui présente le premier coulisseau (10, 61, 101, 131, 161) et un logement de poussoir (63) dans lequel est disposé le poussoir (8) de manière à pouvoir être décalé par rapport à l'agencement de coulisseau (60), dans lequel l'agencement de coulisseau (60) est couplé au premier entraîneur (82, 92, 97) de sorte que le premier entraîneur (82, 92, 97) est couplé au piston (19), et présente une butée (65), le poussoir (8) présente un épaississement de poussoir (66) qui est conçu pour venir en butée contre la butée (65) lorsque le poussoir (8) se décale contre la direction d'introduction (18) par rapport à l'agencement de coulisseau (60).

10. Injecteur selon l'une quelconque des revendications 1 à 9, dans lequel le premier entraîneur (82, 92, 97) est couplé de manière amovible au piston (19) .
